# EUROPEAN PATENT SPECIFICATION

(11) **EP 3 400 919 B1**
(45) Date of publication and mention of the grant of the patent: **19.08.2020**
(21) Application number: 17170950.4
(22) Date of filing: 12.05.2017
(51) Int. Cl.: A61F 13/49, A61F 13/56

(54) **ELASTICISED ABSORBENT ARTICLE**
ELASTISCHER SAUGFÄHIGER ARTIKEL
ARTICLE ABSORBANT ÉLASTIQUE

(43) Date of publication of application: 14.11.2018
(73) Proprietor: Ontex BVBA, 9255 Buggenhout (BE); Ontex Group NV, 9320 Erembodegem (BE)
(72) Inventor: Heege, Thomas, 56761 Düngenheim (DE); Assenheimer, Helen, D-56727 Mayen (DE); Hufnagel, Hans, D-56727 Mayen (DE); Coppejans, Toon, 9320 Aalst-Erembodegem (BE)
(74) Representative: Macchetta, Andrea

(56) References cited:
- WO-A1-2009/157835
- WO-A1-2017/058165

## Description

### TECHNICAL FIELD

The invention pertains to the technical field of absorbent hygiene products. In particular, the present invention relates to an absorbent article for absorbing body fluids and exudates, such as urine and fecal material. More particularly, the present invention relates to absorbent garments, such as disposable diapers for example for babies, which are configured to collect and contain fecal material and avoid leakage.

### BACKGROUND

Disposable diapers conventionally include a chassis having liquid permeable topsheet, a liquid impermeable backsheet and an absorbent structure sandwiched between the topsheet and backsheet. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

As a user of a diaper moves about (i.e. eats, breathes, sneezes, crawls, walks, jumps, etc.), the circumference of the user's waist expands and contracts, which consequently results in the waist portions of the diaper being strained and relaxed. Repeated or exaggerated expansion and contraction of the waist portions can lead to permanent deformation of the waist portions, resulting in a slackening on the diaper around the waist. Particularly for active toddlers wearing diapers which already contain an insult, this often results in the diaper slipping down, thereby increasing the risk for leakage.

To reduce the risk of leakage when worn, a diaper should be provided with form-fitting properties at least in some areas. The form-fitting properties also contribute to an improved appearance of the diaper when worn by the user. Typically, one or both waist portions may contain an elastic waistband. Furthermore, the side panels on which the fastening tabs are provided may display elastic properties.

Over the years, diapers comprising elasticized portions to address the above identified drawbacks have gone through considerable investigation and refinement.

For example, US7252730B2 describes diapers with a continuous elastic waistband positioned between elastic ears with the elastic waistband extending beyond the chassis side edges. However, elastic material is generally more expensive than non-elastic material and in many cases has poor breathability. Hence such arrangements pose further drawbacks on cost and comfort.

Similarly, US8257333B2 describes diapers with a significant portion of the waist circumference being comprised of elastomeric material. Such leading to similar drawbacks as identified above.

In other diapers, such as described in US9358161B2, elastic waistbands are comprised which are located on the edge of the diaper chassis edge. Such however give rise to a reduced anti-leakage performance.

An attempt to solve the above mentioned drawbacks was made with diapers such as described in EP2157958B1, wherein a shortened elastic waistband is positioned in particular such that (i) the distance between an extremity of the diaper chassis and the elastic waistband is less than the distance between the elastic waistband and the core of the diaper, and (ii) the axis running through the tapes of the diaper (when in a fully stretched position) extends between the elastic waistband and the core of the diaper. Although this arrangement effectively reduces the amount of elastic material being used and hence reduces the cost, it does introduce disadvantages in terms of comfort and risk of leakage in case of e.g. sudden liquid stool explosions. Indeed, the specific positioning of the elastic waistband being at a higher position than the elastic ears axis results in torsion of the waist forcing the elastic ears to extend at an angle which results in untensioned areas through which liquid may flow through, particularly when at higher pressure such as in the case of liquid stool explosions, or upon dynamic movement and sudden compression of the diaper such as when a baby drops from a standing position to a sitting position onto the ground.

WO2017058165 relates to an absorbent article including a chassis including an absorbent body. The chassis including a body facing surface and a waist containment member. The waist containment member can include a proximal portion coupled to the body facing surface of the chassis and a distal portion that includes a distal edge. The proximal portion includes proximal portion elastic members and the distal portion can include distal portion elastic members. The distal portion can be free to move with respect to the chassis when the absorbent article is in a relaxed configuration.

WO2009157835 relates to an absorbent article comprising: a chassis extending about a longitudinal axis, a pair of opposed rear elastic side panels attached to said chassis at a rear waist region, a fastening system comprising a first fastening member with first fastening means arranged on each rear elastic side panel and complementary second fastening means, whereby a waist elastic is arranged to extend substantially parallel to a rear transverse edge and spaced therefrom by a second distance and spaced from an absorbent structure by a third distance, said waist elastic terminating short of the longitudinal edges by a fourth distance, wherein said rear elastic side panels and said waist elastic are tailored such that, when said rear waist region is in a substantially maximum extended state, the relative extension of the side panels is approximately the same as or greater than the relative extension of the waist elastic, and wherein said first fastening means of the rear side panels are positioned on a second transverse axis extending substantially parallel to said first transverse axis, with said second transverse axis passing through said waist elastic, such that, during extension of said rear waist region from its relaxed state to a substantially maximum extended state, said waist elastic will reach a substantial proportion of its maximum relative extension before said rear elastic side panels reach the same substantial proportion of their maximum relative extension from their relaxed state.

Thus, there is still need for improvement of the properties of absorbent articles comprising an elastic waistband.

### SUMMARY OF THE INVENTION

The disclosure relates to an absorbent article, preferably a diaper, as described in Claim 1 herein below.

### DESCRIPTION OF FIGURES

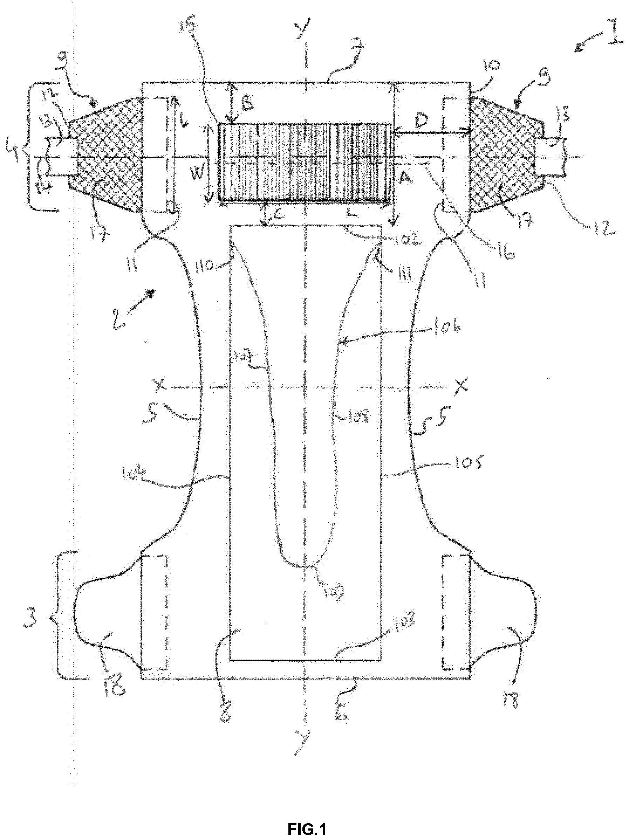

**Fig. 1** is a schematic plan view of an unassembled and fully stretched article according to an embodiment of the present disclosure.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The expression "% by weight" (weight percent), here and throughout the description unless otherwise defined, refers to the relative weight of the respective component based on the overall weight of the formulation.
   The recitation of numerical ranges by endpoints includes all numbers and fractions subsumed within that range, as well as the recited endpoints.
"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.
Preferably, a diaper comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.
The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.
"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.
The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).
As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.
As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.
As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present invention can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present invention. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.
As used therein, the term "associated" encompasses configurations in which top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.
The terms "back section" and "rear back section" are used herein as synonyms and refer to the area of the absorbent article which is contact with the back of the wearer when the absorbent article is worn.
The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.
The terms "belly section" and "front belly section" are used herein as synonyms and refer to the area of the absorbent article which is contact with the belly of the wearer when the absorbent article is worn.
The term "blend" means a mixture of two or more polymers while the term "alloy" means a sub-class of blends wherein the components are immiscible but have been compatibilized.
As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.
"Bonded" refers to the joining, adhering, connecting, attaching, or the like, of at least two elements. Two elements will be considered to be bonded together when they are bonded directly to one another or indirectly to one another, such as when each is directly bonded to intermediate elements.
The term "breathable" refers to layers, preferably films or elastic laminates, having a water vapor transmission rate (WVTR) of at least 300 grams/m² - 24 hours.
"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.
As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.
"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.
"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.
"Compression" refers to the process or result of pressing by applying force on an object, thereby increasing the density of the object.
The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.
Further, the diaper can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.
The term "transversal barriers or flaps" as used herein are essentially pockets formed proximal to and extending along the back and/or front belly waist portions (also referred to as sections) of the diaper. The purpose of such barriers is to collect exudates that flow towards the waist sections of the diaper similarly to the function of the cuffs that prevent leakage through the leg portions of the diaper.
The diaper can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.
"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means as are known in the art.
"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.
The term "density" or "concentration" when referring to the absorbent material, in particular the SAP, of a layer, refers to the amount of the absorbent material divided by the surface area of the layer over which the absorbent material is spread out.
As used herein, the term "diaper" refers to an absorbent article generally worn by a user (preferably a baby, toddler and/or children) about the lower torso.
"Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.
The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).
As used herein, the term "elastic resistance" describes an elastic force that tends to resist an applied tensile force causing a material provided therewith to tend to contract to an untensioned configuration in response to a stretching force. The elastic resistance may be measured by use of a testcontrol II Zugprüfmaschine (available at ZWICK GmbH & Co. KG), clamping the opposite ends of the intended component (e.g. the ear or the elastic waistband) with respective clamp members connected at opposite first and second ends of the testcontrol II, and apply a displacement of 1 cm/s moving said first and second ends in opposed directions. The force is recorded during the displacement, with the force required to reach a stretch of 10% being taken as the elastic resistace.
As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.
The term "elasticized" or "elastified" refers to a material, layer, or substrate that is naturally non-elastic, but which has been rendered elastic by, for example, suitably joining an elastic material, layer, or substrate thereto.
"Elongation" means the ratio of the extension of a material to the length of the material prior to the extension (expressed in percent). "Extension" means the change in length of a material due to stretching (expressed in units of length).
As used herein the term "extensible" means elongatable in at least one direction, but not necessarily recoverable.
The term "fabrics" is used to refer to all of the woven, knitted and nonwoven fibrous webs.
The term "finished" or "final", when used with reference to a product, means that the product has been suitably manufactured for its intended purpose.
The term "flexible" refers to materials which are compliant and which will readily conform to the general shape and contours of the wearer's body.
As used herein, the term "garment" means any type of apparel which may be worn. This includes diapers, training pants, incontinence products, surgical gowns, industrial workwear and coveralls, undergarments, pants, shirts, jackets and the like.
The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.
The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alpha-olefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.
"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.
The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.
As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.
"Integral" is used to refer to various portions of a single unitary element rather than separate structures bonded to or placed with or placed near one another.
"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.
The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer (i.e. typically in a manner as illustrated in Fig.1 to Fig.5).
"Laminate" refers to elements being attached together in a layered arrangement.
The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.
"Liquid" means a nongaseous substance and/or material that flows and can assume the interior shape of a container into which it is poured or placed.
"Longitudinal" is a direction running parallel to the maximum linear dimension of the article.
The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.
The term "nonelastic" or "inelastic" refers to any material which does not fall within the definition of "elastic" above, typically by not comprising elastic materials such as elastic web materials described herein.
The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.
By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.
The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.
"Pulp fluff" or "fluff pulp" refers to a material made up of cellulose fibers. The fibers can be either natural or synthetic, or a combination thereof. The material is typically lightweight and has absorbent properties.
The "retention portion" or "liquid absorption layer" is part of the absorbent medium (or core). This portion may comprise a matrix of hydrophilic fibers, such as a web of cellulosic fluff, mixed with particles of high-absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.
As used herein the term "sheet" or "sheet material" refers to woven materials, nonwoven webs, polymeric films, polymeric scrim-like materials, and polymeric foam sheeting.
The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.
The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.
"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.
By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bi-directional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.
Use of the term "substrate" includes, but is not limited to, woven or nonwoven webs, porous films, ink permeable films, paper, composite structures, or the like.
Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight. Typically, the superabsorbent material, when present, will be included in an amount of about 5% to about 40% by weight, based on the total weight of the absorbent layer.
"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.
"Tension" includes a uniaxial force tending to cause the extension of a body or the balancing force within that body resisting the extension.
As used herein, the term "thermoplastic" is meant to describe a material that softens when exposed to heat and which substantially returns to its original condition when cooled to room temperature.
The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.
As used herein, the term "transverse" or "lateral" refers to a line, axis, or direction which lies within the plane of the absorbent article and is generally perpendicular to the longitudinal direction.
"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.
As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

Embodiments of the articles according to the disclosure will now be described. It is understood that technical features described in one or more embodiments maybe combined with one or more other embodiments without departing from the intention of the disclosure and without generalization therefrom.

With reference to Fig. 1, the absorbent article (1), preferably a diaper, having a longitudinal axis (Y-Y) and a transverse axis (X-X), comprises: a chassis (2) including a skin-facing side, a garment-facing side opposite to the skin-facing side, a front waist region (3), a back waist region (4), a crotch region (5) extending between said front and back waist regions (3,4) and opposed front and back transverse edges (6,7) positioned on the front and back waist regions (3.4) respectively, said chassis comprising a topsheet, a backsheet and an absorbent core (8) disposed between said topsheet and said backsheet, said topsheet being arranged on said skin-facing side, said backsheet being arranged on said garment-facing side, and said absorbent core (8) extending through at least a portion of the crotch region (5), and wherein said absorbent core (8) terminates at a first distance (A) from said back transverse edge (7); a pair of opposed elasticized side panels (9) joined to the chassis at the back waist region (4), each side panel extending out from respective chassis longitudinal edges (10) of the chassis at the back waist region (4), wherein each said side panel (9) comprises first and second longitudinal edges (11,12) oppositely disposed along an axis parallel to the transverse axis (X-X) with the first longitudinal edge (11) being longer than a second longitudinal edge (12), said elasticized side panels (9) being joined to the chassis (2) along said first longitudinal edge (11); one or more fastening members (13) joined to each of said opposed elasticized side panels (9) releasably joinable to a garment facing side of the front waist region (3), wherein opposing fastening members (13) are positioned on a fastener axis (14) extending parallel to the transverse axis (X-X); and an elastic waistband member (15) positioned on said back waist region (4) between said pair of opposed elasticized side panels (9) and extending along an axis parallel to the transverse axis (X-X), wherein said elastic waistband member (15) is positioned at a second distance (B) from said back transverse edge (7) and a third distance (C) from the absorbent core (8), said elastic waistband member (15) terminating short of each opposed chassis longitudinal edges (10) by a fourth distance (D), and wherein said elastic waistband member (15) has a width (W) extending parallel to said longitudinal axis (Y-Y) and a length (L) extending parallel to said transverse axis (X-X) and said back transverse edge (7). The second distance (B) being greater than the third distance (C) and wherein said width (W) is greater than 25% of the length (I) of said first longitudinal edge (11). An advantage of this arrangement is improved comfort and adherence onto the wearer's torso that further aids in reducing the risk of leakage particularly when a subject is rolling on the ground or sudden drop.

Optionally, the absorbent article may comprise frontal side panels (18) which are joined to the chassis by an adhesive. The frontal side panels (18) are preferably inelastic.

In an embodiment, the second distance (B) is 25%, preferably 30%, more preferably 40%, greater than the third distance (C), most preferably the second distance (B) being from 30% to 95% greater than the third distance (C).

In an embodiment, the width (W) is between 30% and 100%, preferably between 31% and 90%, more preferably between 35% and 80%, even more preferably from 50% to 80%, of the length (I) of said first longitudinal edge (11).

The elastic waistband member may be extended from its first, relaxed length to a second, extended length. The elastic waistband member may be applied in its second, extended length to absorbent article. When the elastic waistband member is allowed to relax, the absorbent article comprising the elastic waistband member may contract to approximately the first, relaxed length of the elastic waistband member. Gathers or pleats may be formed in or adjacent to the elastic waistband member of absorbent article. The elastic waistband may be applied onto the topsheet or may be applied to other components of absorbent article, such as backsheet, or may be sandwiched between different layers comprising absorbent article, such as between the topsheet and backsheet.

In some embodiments, the elastic waistband member is constructed by stretching an elastic material and, while the elastic material is in a stretched state, bonding the elastic material to at least one precursor web in such a manner that, when tension is released on the resulting elastic waistband member, the entire elastic waistband member contracts. The elastic material may have a first, relaxed length, and a second, extended length. The second, extended length does not need to be, but may be, a fully extended length. The elastic material may be applied at its second, extended length to precursor web while precursor web is in a relaxed state, or is under tension sufficient to reduce slack in precursor web without extending precursor web. When the elastic material is allowed to relax, the elastic waistband member comprising elastic material and precursor web will contract to approximately the first, relaxed length of the elastic material (with some variance based on the elastic recovery of elastic material from its second, extended length and because the finite bending stiffness of the joined materials may prevent full contraction). Gathers or pleats may be formed in the elastic waistband member.

Precursor web or webs may comprise nonwovens, films, or composites thereof, and may each, for example, have a basis weight in the range of 10 gsm to 30 gsm. If more than one precursor web is present, the webs may be the same or different in composition, form (i.e., woven, nonwoven, films, etc.), and basis weight. The elastic material may comprise an elastomeric film or one or more elastomeric strands or ribbons. The relative amount that elastic material is stretched prior to combining to form the elastic waistband member may be referred to as "installed elongation". For example, if the first, relaxed dimension of the elastic material is 100mm and the elastic material is stretched by 85 mm to a second, extended length of 185 mm prior to combining the elastic material with the precursor web to form the elastic waistband member, the installed elongation is 85%.

The installed elongation may be different or substantially the same as than the applied elongation of the elastic waistband member to the garment or absorbent article. The "applied elongation" refers to the relative amount the elastic waistband member is stretched versus its first, relaxed dimension prior to joining, bonding or affixing it to the garment or absorbent article. For example, if a piece of the elastic waistband member as described above, having a 85% installed elongation, has a first, relaxed dimension of 100mm and is elongated 55mm prior to attachment to a garment or absorbent article, the elastic waistband member has an applied elongation of 55%. The relative amount of contraction this elastic waistband member will cause in the region of the garment to which it is affixed may vary, depending on the relative stiffness of the garment. If the garment is very flexible in the waistband region, the amount of contraction may approach 35%. However, if the garment is very stiff in that region, the amount of contraction of the garment may be significantly less than 35%.

In an embodiment, the pair of opposed elasticized side panels (9) have a first and second installed elongation, and the elastic waistband member (15) has a third installed elongation, wherein the first and second installed elongations are substantially the same and are less than the third installed elongation. An advantage of this arrangement is to provide modulated stretch between the elastic waistband and the elastic panels.

In an embodiment, the third installed elongation is 20%, preferably 25%, more preferably 30%, greater than the first installed elongation, even more preferably wherein the third installed elongation is from 35% to 80%, greater than the first installed elongation.

In an embodiment, the elastic waistband member (15) has an applied elongation that is graded such that it varies along the length (L) thereof.

In an embodiment, the elastic waistband member (15) has an applied elongation that is from 15% to 70%, preferably from 20% to 55%, of the installed elongation.

In an embodiment, the pair of opposed elasticized side panels (9) have a first and second elastic resistance, and the elastic waistband member (15) has a third elastic resistance, wherein the first and second elastic resistance are substantially the same and are greater than the third elastic resistance. An advantage of this embodiment is that when the user is applying the product by stretching the elastic ears, it first stretches the elastic waistband up to its maximum elongation prior to completely stretching the elastic ears and hence ensures an even better fit particularly on the back portion of the diaper, this also in turn may result in improved resistance to leakage.

In a preferred embodiment, the elastic waistband member (15) is positioned on the fastener axis (14), such that when the absorbent article (1) is in extended state, the fastener axis (14) crosses the elastic waistband member (15). Such arrangement has the advantage to reduce side panel bending and hence ensure a more uniform fit and improved barrier to leakage. Moreover, it has been surprisingly found that such positioning of the elastic enables to increase the stretchability of the fastening system by more than 5%, typically about 16% (at 30 N) as compared to the same diaper but with the elastic waistband member positioned offset from the fastener axis (i.e. not on the fastener axis).

In a particularly preferred embodiment, the elastic waistband member (15) has a centerline axis (16) dividing the elastic waistband member (15) into two equal halves and extending parallel to the transverse axis (X-X), wherein said centerline axis (16) is offset (i.e. adjacent to, but not in-line with) from the fastener axis (14), preferably wherein the centerline axis (16) is positioned at a first axial distance away from said fastener axis (14) along the longitudinal axis (Y-Y). An advantage of this embodiment is to provide the above mentioned reduced side panel bending and hence ensure a more uniform fit and improved barrier to leakage, but at the same time add an additional resistance that enables the elastic side panels to be fully stretched upon fitting and thus limit the risk of the elastic waistband to become fully stretched without the side panels also being fully stretched. Moreover a more even stretch on first-time-use may be achieved.

Preferably, the first axial distance does not exceed 45%, preferably 40%, more preferably 30%, even more preferably 25%, of the width (W).

In an embodiment (not shown), the absorbent article may comprise at least one transversal barrier cuff positioned on the back waist region and extending parallel to the elastic waistband member length. The barrier cuff may form a pocket for collection of exudates therein and may comprise one or more elastics positioned at an open end thereof being distal from a closed end thereof. Preferably, the barrier cuff is positioned below the elastic waistband member on the skin facing surface of the absorbent article. In this arrangement, the elastic waistband member provides for a tight closure at a closed end of the pocket formed by the transverse barrier aiding the effectiveness of the leakage prevention barrier.

The absorbent core (8) comprises: a core length extending along the longitudinal axis (Y-Y), a core width extending perpendicular to said core length and a perimeter comprising at least two opposing ends (102, 103) and at least two opposing sides (104, 105) positioned between said ends (102, 103) wherein the absorbent core (8) comprises one or more substantially interconnected channels (106) extending through the crotch region (5) along and substantially parallel to the longitudinal axis and further extending along at least a portion of said core width from one side of the core (8) to the other, preferably said one or more substantially interconnected channels (106) being symmetric or asymmetric about the longitudinal axis, wherein each substantially interconnected channel (106) comprises: a first channel portion (107) extending substantially along the longitudinal axis (Y-Y) proximal to a first side (104) of the core (101); a second channel portion (108) extending substantially along the longitudinal axis proximal to a second side (105) of the core (8); and at least one, preferably only one, connecting channel portion (109) in fluid communication with said first and second channel portions (107, 108). An advantage of this arrangement is that better fit can be achieved as well as faster liquid distribution over the core, which adds and complements the leakage resistance capabilities of the absorbent article.

The core typically comprises one or more absorbent materials such as cellulosic fibers and/or super absorbent polymers (in the form of particles, flakes and/or fibers). Preferably, the channels are substantially free of absorbent material and are typically formed by two nonwoven layers joined together, typically by an adhesive.

The connecting channel portion (109) extends substantially along the width of said core (8), preferably forming a closed end shape within a surface of said core (8) along a plane parallel to the longitudinal axis, and preferably positioned opposite to an open end shape formed by non-connected first and second terminal positions (110,111) of the interconnected channel (106), preferably of the first and second channel portions (107, 108) respectively, typically said non-connected first and second terminal positions (110, 111) being distal to each other and proximal to the first and second sides (104, 105) of said core (8) respectively, even more preferably said terminal positions (110, 111) facing away from each other such to form a funnel-shaped gap therebetween. Such geometry further promotes the formation of a "cup-shape" at the crotch region as well as optimal liquid distribution across the length and width of the absorbent core.

The first and second terminal positions (110, 111) are arranged at a second axial distance from the elastic waistband member (15), said second axial distance being less than or substantially equal to said second distance (B). This arrangement ensures greater resistance to leakage provided by the adhering force generated by the elastic waistband.

In an embodiment, the pair of opposed elasticized side panels (9) are joined to the chassis via an adhesive and/or by mechanical bonding such as ultrasonic bonding. In this embodiment, the elastic panels (9) comprise an elastic film (17) typically interposed (or laminated) between two nonwoven layers wherein the perimeter of the elastic film is less than the total perimeter of the elastic panels (9) such to form an area of the side panel (9) free of said elastic film (17) typically wherein said area free of elastic film is proximal to the portion of the side panel (9) being joined to the chassis. In a preferred embodiment at least a portion of the mechanical bond contacts the elastic film (17) and the area free of said film. An advantage of this arrangement is to further increase the joining force of the side panels to the chassis when used in combination with an elastic waistband.

It is supposed that the present invention is not restricted to any form of realization described previously and that some modifications can be added to the presented example of fabrication without reappraisal of the appended claims.

## Claims

1. An absorbent article (1), preferably a diaper, having a longitudinal axis (Y-Y) and a transverse axis (X-X), comprising:
a chassis (2) including a skin-facing side, a garment-facing side opposite to the skin-facing side, a front waist region (3), a back waist region (4), a crotch region (5) extending between said front and back waist regions (3,4) and opposed front and back transverse edges (6,7) positioned on the front and back waist regions (3.4) respectively, said chassis comprising a topsheet, a backsheet and an absorbent core (8) disposed between said topsheet and said backsheet, said topsheet being arranged on said skin-facing side, said backsheet being arranged on said garment-facing side, and said absorbent core (8) extending through at least a portion of the crotch region (5), and wherein said absorbent core (8) terminates at a first distance (A) from said back transverse edge (7);
a pair of opposed elasticized side panels (9) joined to the chassis at the back waist region (4), each side panel extending out from respective chassis longitudinal edges (10) of the chassis at the back waist region (4), wherein each said side panel (9) comprises first and second longitudinal edges (11,12) oppositely disposed along an axis parallel to the transverse axis (X-X) with the first longitudinal edge (11) being longer than a second longitudinal edge (12), said elasticized side panels (9) being joined to the chassis (2) along said first longitudinal edge (11);
one or more fastening members (13) joined to each of said opposed elasticized side panels (9) releasably joinable to a garment facing side of the front waist region (3), wherein opposing fastening members (13) are positioned on a fastener axis (14) extending parallel to the transverse axis (X-X);
an elastic waistband member (15) positioned on said back waist region (4) between said pair of opposed elasticized side panels (9) and extending along an axis parallel to the transverse axis (X-X), wherein said elastic waistband member (15) is positioned at a second distance (B) from said back transverse edge (7) and a third distance (C) from the absorbent core (8), said elastic waistband member (15) terminating short of each opposed chassis longitudinal edges (10) by a fourth distance (D), and wherein said elastic waistband member (15) has a width (W) extending parallel to said longitudinal axis (Y-Y) and a length (L) extending parallel to said transverse axis (X-X) and said back transverse edge (7);
wherein said second distance (B) is greater than said third distance (C) and said width (W) is greater than 25% of the length (I) of said first longitudinal edge (11), **characterized in that** said second distance (B) is from 30% to 95% greater than the third distance (C), and wherein the absorbent core (8) comprises: a core length extending along the longitudinal axis (Y-Y), a core width extending perpendicular to said core length and a perimeter comprising at least two opposing ends (102, 103) and at least two opposing sides (104, 105) positioned between said ends (102, 103) wherein the absorbent core (8) comprises one or more substantially interconnected channels (106) extending through the crotch region (5) along and substantially parallel to the longitudinal axis and further extending along at least a portion of said core width from one side of the core (8) to the other, wherein each substantially interconnected channel (106) comprises: a first channel portion (107) extending substantially along the longitudinal axis (Y-Y) proximal to a first side (104) of the core (101); a second channel portion (108) extending substantially along the longitudinal axis proximal to a second side (105) of the core (8); and at least one connecting channel portion (109) in fluid communication with said first and second channel portions (107, 108), and wherein the connecting channel portion (109) extends substantially along the width of said core (8) forming a closed end shape within a surface of said core (8) along a plane parallel to the longitudinal axis and positioned opposite to an open end shape formed by non-connected first and second terminal positions (110,111) of the interconnected channel (106), wherein the first and second terminal positions (110, 111) are arranged at a second axial distance from the elastic waistband member (15), said second axial distance being less than or equal to said second distance (B).

2. An absorbent article (1) according to Claim 1 wherein the width (W) is between 30% and 100%, preferably between 31% and 90%, more preferably between 35% and 80%, even more preferably from 50% to 80%, of the length (I) of said first longitudinal edge (11).

3. An absorbent article (1) according to any of the preceding Claims wherein the pair of opposed elasticized side panels (9) have a first and second installed elongation, and the elastic waistband member (15) has a third installed elongation, wherein the first and second installed elongations are substantially the same and are less than the third installed elongation.

4. An absorbent article (1) according to Claim 3 wherein the third installed elongation is 20%, preferably 25%, more preferably 30%, greater than the first installed elongation, even more preferably wherein the third installed elongation is from 35% to 80%, greater than the first installed elongation.

5. An absorbent article (1) according to any of the preceding Claims wherein the elastic waistband member (15) has an applied elongation that is graded such that it varies along the length (L) thereof.

6. An absorbent article (1) according to Claims 3 to 5 wherein elastic waistband member (15) has an applied elongation that is from 15% to 70%, preferably from 20% to 55%, of the installed elongation.

7. An absorbent article (1) according to any of the preceding Claims wherein the elastic waistband member (15) is positioned on the fastener axis (14), such that when the absorbent article (1) is in extended state, the fastener axis (14) crosses the elastic waistband member (15).

8. An absorbent article (1) according to Claim 7 wherein the elastic waistband member (15) has a centerline axis (16) dividing the elastic waistband member (15) into two equal halves and extending parallel to the transverse axis (X-X), wherein said centerline axis (16) is adjacent to, but not in-line with, the fastener axis (14), preferably wherein the centerline axis (16) is positioned at a first axial distance away from said fastener axis (14) along the longitudinal axis (Y-Y).

9. An absorbent article (1) according to Claim 8 wherein the first axial distance does not exceed 45%, preferably 40%, more preferably 30%, even more preferably 25%, of the width (W).

10. An absorbent article (1) according to any of the preceding Claims wherein the one or more substantially interconnected channels (106) are symmetric or asymmetric about the longitudinal axis, and preferably comprises only one connecting channel portion (109) in fluid communication with said first and second channel portions (107, 108).

11. An absorbent article (1) according to Claim 10 wherein the connecting channel portion (109) extends substantially along the width of said core (8), preferably forming a closed end shape within a surface of said core (8) along a plane parallel to the longitudinal axis, and preferably positioned opposite to an open end shape formed by non-connected first and second terminal positions (110,111) of the first and second channel portions (107, 108) of the interconnected channel (106) respectively, said non-connected first and second terminal positions (110, 111) being distal to each other and proximal to the first and second sides (104, 105) of said core (8) respectively, preferably said terminal positions (110, 111) facing away from each other such to form a funnel-shaped gap therebetween.

12. An absorbent article (1) according to any of the preceding claims wherein the pair of opposed elasticized side panels (9) have a first and second elastic resistance, and the elastic waistband member (15) has a third elastic resistance, wherein the first and second elastic resistance are substantially the same and are greater than the third elastic resistance.

13. An absorbent article (1) according to any of the preceding Claims wherein the pair of opposed elasticized side panels (9) are joined to the chassis via an adhesive and/or by mechanical bonding such as ultrasonic bonding.

## Patentansprüche

1. Saugfähiger Artikel (1), vorzugsweise eine Windel mit einer Längsachse (Y - Y)und einer Querachse (X - X), umfassend:
einen Körper (2), darin eingeschlossen eine zur Haut gerichtete Seite, eine zur Kleidung gerichtete Seite gegenüber der zur Haut gerichteten Seite, eine vordere Hüftregion (3), eine hintere Hüftregion (4), eine Schrittregion (5), die sich zwischen der vorderen und hinteren Hüftregion (3, 4) erstreckt, und gegenüberliegende vordere und hintere Querkanten (6, 7), die auf den vorderen bzw. hinteren Hüftregionen (3, 4) positioniert sind, wobei der Körper eine obere Schicht, eine hintere Schicht und einen saugfähigen Kern (8) umfasst, der zwischen der oberen Schicht und der hinteren Schicht angeordnet ist, wobei die obere Schicht auf der zur Haut gerichteten Seite angeordnet ist, wobei die hintere Schicht auf der zur Kleidung gerichteten Seite angeordnet ist, wobei sich der saugfähige Kern (8) durch mindestens einen Abschnitt der Schrittregion (5) ertreckt, und wobei der saugfähige Kern (8) in einem ersten Abstand (A) von der hinteren Querkannte (7) endet;
ein Paar einander gegenüberliegender elastischer Seitenteile (9), die mit dem Körper an der hinteren Hüftregion (4) verbunden sind, wobei sich jeder Seitenteil von entsprechenden Körperlängskanten (10) des Körpers an der hinteren Hüftregion (4) nach außen erstreckt, wobei jeder Seitenteil (9) erste und zweite Längskanten (11, 12) umfasst, die einander gegenüberliegend entlang einer Achse parallel zur Querachse (X - X) angeordnet sind, wobei die erste Längskante (11) länger als eine zweite Längskante (12) ist, wobei die elastischen Seitenteile (9) an den Körper (2) entlang der ersten Längskante (11) verbunden sind,
ein oder mehrere Befestigungselemente (13) mit jedem der einander gegenüber liegenden elastischen Seitenteile (9) verbunden sind, die abnehmbar mit einer zur Kleidung gerichteten Seite der vorderen Hüftregion (3) verbunden werden können, wobei einander gegenüberliegende Befestigungselemente (13) auf einer Befestigungsachse (14) positioniert sind, die sich parallel zur Querachse (X - X) erstreckt;
ein elastisches Hüftbandelement (15), das auf der hinteren Hüftregion (4) zwischen dem Paar einander gegenüberliegender elastischer Seitenteile (9) positioniert ist und sich entlang einer Achse parallel zur Querachse (X - X) erstreckt, wobei das elastische Hüftbandelement (15) in einem zweiten Abstand (B) von der hinteren Querkante (7) und einem dritten Abstand (C) von dem saugfähigen Kern (8) positioniert ist, wobei das elastische Hüftbandelement (15) kurz vor jeder gegenüberliegenden Körperlängskante (10) in einem vierten Abstand (D) endet, und wobei das elastische Hüftbandelement (15) eine Breite (W) aufweist, die sich parallel zu der Längsachse (Y - Y) erstreckt, und eine Länge (L), die sich parallel zu der Querachse (X - X) und der hinteren Querkante (7) erstreckt;
wobei der zweite Abstand (B) größer als der dritte Abstand (C) ist und die Breite (W) größer als 25 % der Länge (I) der ersten Längskante (11) ist, **dadurch gekennzeichnet, dass** der zweite Abstand (B) von 30 % bis 95 % größer als der dritte Abstand (C) ist, und wobei der saugfähige Kern (8) Folgendes umfasst: eine Kernlänge, die sich entlang der Längsachse (Y - Y) erstreckt, eine Kernbreite, sie sich senkrecht zur der Kernlänge erstreckt, und einen Umfang, der mindestens zwei einander gegenüberliegende Enden (102, 103) und mindestens zwei einander gegenüberliegende Seiten (104, 105) umfasst, die zwischen den Enden (102, 103) positioniert sind, wobei der saugfähige Kern (8) einen oder mehrere im Wesentlichen miteinander verbundene Kanäle (106) umfasst, die sich durch den Schrittbereich (5) entlang und im Wesentlichen parallel zur Längsachse erstrecken und sich weiter entlang mindestens eines Abschnitts der Kernbreite von einer Seite des Kerns (8) zur anderen erstrecken, wobei jeder im Wesentlichen verbundene Kanal (106) Folgendes umfasst: einen ersten Kanalabschnitt (107), der sich im Wesentlichen entlang der Längsachse (Y - Y) proximal zu einer ersten Seite (104) des Kerns (101) erstreckt; einen zweiten Kanalabschnitt (108), der sich im Wesentlichen entlang der Längsachse proximal zu einer zweiten Seite (105) des Kerns (8) erstreckt; und mindestens einen verbindenden Kanalabschnitt (109) in fluidischer Kommunikation mit dem ersten und dem zweiten Kanalabschnitt (107, 108) und wobei sich der verbindende Kanalabschnitt (109) im Wesentlichen entlang der Breite des Kerns (8) erstreckt, die eine geschlossene Endform innerhalb einer Fläche des Kerns (8) entlang einer Ebene parallel zur Längsachse bildet und gegenüber einer offenen Endform positioniert ist, die von nicht verbundenen ersten und zweiten terminalen Positionen (110, 111) des verbundenen Kanals (106) gebildet ist, wobei die erste und zweite terminale Position (110, 111) in einem zweiten axialen Abstand vom elastischen Hüftbandelement (15) angeordnet ist, wobei der zweite axiale Abstand kleiner als oder gleich wie der zweite Abstand (B) ist.

2. Saugfähiger Artikel (1) nach Anspruch 1, wobei die Breite (W) zwischen 30 % und 100 %, vorzugsweise zwischen 31 % und 90 %, insbesondere zwischen 35 % und 80 %, noch bevorzugter von 50 % bis 80 % der Länge (I) der ersten Längskante (11) beträgt.

3. Saugfähiger Artikel (1) nach einem der vorhergehenden Ansprüche, wobei das Paar einander gegenüberliegender elastischer Seitenteile (9) eine erste und zweite installierte Verlängerung aufweist und das elastisches Hüftbandelement (15) eine dritte installierte Verlängerung aufweist, wobei die erste und zweite installierte Verlängerung im Wesentlichen gleich wie und kleiner als die dritte installierte Verlängerung sind.

4. Saugfähiger Artikel (1) nach Anspruch 3, wobei die dritte installierte Verlängerung 20 %, vorzugsweise 25 %, insbesondere 30 % größer als die erste installierte Verlängerung ist, noch bevorzugter wobei die dritte installierte Verlängerung von 35 % bis 80 % größer als die erste installierte Verlängerung ist.

5. Saugfähiger Artikel (1) nach einem der vorhergehenden Ansprüche, wobei das elastisches Hüftbandelement (15) eine angebrachte Verlängerung aufweist, die derart abgestuft ist, dass sie entlang der Länge (L) davon variiert.

6. Saugfähiger Artikel (1) nach Anspruch 3 bis 5, wobei das elastische Hüftbandelement (15) eine aufgebrachte Verlängerung aufweist, die von 15 % bis 70 %, vorzugsweise von 20 % bis 55 % der installierten Verlängerung beträgt.

7. Saugfähiger Artikel (1) nach einem der vorhergehenden Ansprüche, wobei das elastische Hüftbandelement (15) auf der Befestigungsachse (14) positioniert ist, so dass, wenn sich der saugfähige Artikel (1) im erweiterten Zustand befindet, die Befestigungsachse (14) das elastische Hüftbandelement (15) kreuzt.

8. Saugfähiger Artikel (1) nach Anspruch 7, wobei das elastische Hüftbandelement (15) eine Mittellinienachse (16) aufweist, die das elastische Hüftbandelement (15) in zwei gleiche Hälften teilt und sich parallel zur Querachse (X - X) erstreckt, wobei die Mittellinienachse (16) benachbart zu, jedoch nicht linear mit der Befestigungsachse (14) ist, vorzugsweise wobei die Mittellinienachse (16) in einem ersten axialen Abstand weg von der Befestigungsachse (14) entlang der Längsachse (Y - Y) ist.

9. Saugfähiger Artikel (1) nach Anspruch 8, wobei der erste axiale Abstand 45 %, vorzugsweise 40 %, insbesondere 30 %, noch bevorzugter 25 % der Breite (W) nicht übersteigt.

10. Saugfähiger Artikel (1) nach einem der vorhergehenden Ansprüche, wobei der eine oder die mehreren im Wesentlichen miteinander verbundene Kanäle (106) symmetrisch oder asymmetrisch um die Längsachse sind, und vorzugsweise nur einen verbindenden Kanalabschnitt (109) in fluidischer Kommunikation mit dem ersten und dem zweiten Kanalabschnitt (107, 108) umfassen.

11. Saugfähiger Artikel (1) nach Anspruch 10, wobei sich der verbindende Kanalabschnitt (109) im Wesentlichen entlang der Breite des Kerns (8) erstreckt, vorzugsweise eine geschlossene Endform innerhalb einer Fläche des Kerns (8) entlang einer Ebene parallel zur Längsachse bildet und vorzugsweise gegenüber einer offenen Endform positioniert ist, die von nicht verbundenen ersten und zweiten terminalen Positionen (110, 111) des ersten bzw. zweiten Kanalabschnitts (107, 108) des verbundenen Kanals (106) gebildet ist, wobei die nicht verbundenen ersten und zweiten terminalen Positionen (110, 111) distal zueinander und proximal zur ersten bzw. zweiten Seite (104, 105) des Kerns (8) sind, vorzugsweise wobei die terminalen Positionen (110, 111) voneinander weg zeigen, so dass sie einen trichterförmigen Zwischenraum dazwischen bilden.

12. Saugfähiger Artikel (1) nach einem der vorhergehenden Ansprüche, wobei das Paar einander gegenüberliegender elastischer Seitenteile (9) einen ersten und zweiten elastischen Widerstand aufweist und das elastische Hüftbandelement (15) einen dritten elastischen Widerstand aufweist, wobei der erste und zweite elastische Widerstand im Wesentlichen gleich wie und größer als der dritte elastische Widerstand ist.

13. Saugfähiger Artikel (1) nach einem der vorhergehenden Ansprüche, wobei das Paar einander gegenüberliegender elastischer Seitenteile (9) mit dem Körper über eine klebende und/oder mechanische Verbindung wie z. B. eine Ultraschallverbindung verbunden sind.

## Revendications

1. Article absorbant (1) de préférence une couche, ayant un axe longitudinal (Y-Y) et un axe transversal (X-X) comprenant :
un châssis (2) comprenant un côté orienté vers la peau, un côté orienté vers le vêtement opposé au côté orienté vers la peau, une région de taille avant (3), une région de taille arrière (4), une région d'entrejambes (5) s'étendant entre lesdites régions de taille avant et arrière (3, 4) et des bords transversaux avant et arrière (6, 7) opposés positionnés sur les régions de taille avant et arrière (3, 4) respectivement, ledit châssis comprenant une feuille supérieure, une feuille inférieure et un cœur absorbant (8) disposé entre ladite feuille supérieure et ladite feuille inférieure, ladite feuille supérieure étant agencée sur ledit côté orienté vers la peau, ladite feuille inférieure étant agencée sur ledit côté orienté vers le vêtement, et ledit cœur absorbant (8) s'étendant à travers au moins une partie de la région d'entrejambes (5) et dans lequel ledit cœur absorbant (8) se termine à une première distance (A) dudit bord transversal arrière (7) ;
une paire de panneaux latéraux élastiques (9) opposés assemblés au châssis au niveau de la région de taille arrière (4), chaque panneau latéral s'étendant à partir des bords longitudinaux de châssis (10) respectifs du châssis au niveau de la région de taille arrière (4), dans lequel chacun desdits panneaux latéraux (9) comprend des premier et second bords longitudinaux (11, 12) disposés à l'opposé le long d'un axe parallèle à l'axe transversal (X-X) avec le premier bord longitudinal (11) qui est plus long qu'un second bord longitudinal (12), lesdits panneaux latéraux élastiques (9) étant assemblés au châssis (2) le long dudit premier bord longitudinal (11) ;
un ou plusieurs éléments de fixation (13) assemblés à chacun desdits panneaux latéraux élastiques (9) opposés pouvant être assemblés de manière amovible à un côté orienté vers le vêtement de la région de taille avant (3), dans lequel les éléments de fixation (13) opposés sont positionnés sur un axe de fixation (14) s'étendant parallèlement à l'axe transversal (X-X) ;
un élément de ceinture élastique (15) positionné sur ladite région de taille arrière (4) entre ladite paire de panneaux latéraux élastiques (9) opposés et s'étendant le long d'un axe parallèle à l'axe transversal (X-X), dans lequel ledit élément de ceinture élastique (15) est positionné à une deuxième distance (B) dudit bord transversal arrière (7) et à une troisième distance (C) du cœur absorbant (8), ledit élément de ceinture élastique (15) s'arrêtant à une courte distance de chacun des bords longitudinaux de châssis (10) opposés selon une quatrième distance (D), et dans lequel ledit élément de ceinture élastique (15) a une largeur (W) s'étendant parallèlement audit axe longitudinal (Y-Y) et une longueur (L) s'étendant parallèlement audit axe transversal (X-X) et audit bord transversal arrière (7) ;
dans lequel ladite deuxième distance (B) est supérieure à ladite troisième distance (C) et ladite largeur (W) est supérieure à 25‰ de la longueur (1) dudit premier bord longitudinal (11), **caractérisé en ce que** ladite deuxième distance (B) est 30% à 95% supérieure à la troisième distance (C), et dans lequel le cœur absorbant (8) comprend : une longueur de cœur s'étendant le long de l'axe longitudinal (Y-Y), une largeur de cœur s'étendant perpendiculairement à ladite longueur de cœur et un périmètre comprenant au moins deux extrémités opposées (102, 103) et au moins deux côtés opposés (104, 105) positionnés entre lesdites extrémités (102, 103), dans lequel le cœur absorbant (8) comprend un ou plusieurs canaux (106) sensiblement interconnectés s'étendant à travers la région d'entrejambes (5) le long de et sensiblement parallèlement à l'axe longitudinal et s'étendant en outre le long d'au moins une partie de ladite largeur de cœur à partir d'un côté du cœur (8) à l'autre, dans lequel chaque canal (106) sensiblement interconnecté comprend : une première partie de canal (107) s'étendant sensiblement le long de l'axe longitudinal (Y-Y) à proximité d'un premier côté (104) du cœur (101) ; une seconde partie de canal (108) s'étendant sensiblement le long de l'axe longitudinal à proximité d'un second côté (105) du cœur (8) ; et au moins une partie de canal de raccordement (109) en communication de fluide avec lesdites première et seconde parties de canal (107, 108) et dans lequel la partie de canal de raccordement (109) s'étend sensiblement le long de la largeur dudit cœur (8) formant une forme d'extrémité fermée avec une surface dudit cœur (8) le long d'un plan parallèle à l'axe longitudinal et positionnée à l'opposé d'une forme d'extrémité ouverte formée par les première et seconde positions terminales (110, 111) non raccordées du canal (106) interconnecté, dans lequel les première et seconde positions terminales (110, 111) sont agencées à une deuxième distance axiale par rapport à l'élément de ceinture élastique (15), ladite deuxième distance axiale étant inférieure ou égale à ladite deuxième distance (B).

2. Article absorbant (1) selon la revendication 1, dans lequel la largeur (W) est comprise entre 30% et 100%, de préférence entre 31% et 90%, encore de préférence entre 35% et 80%, encore de préférence de 50% à 80% de la longueur (1) dudit premier bord longitudinal (11).

3. Article absorbant (1) selon l'une quelconque des revendications précédentes, dans lequel la paire de panneaux latéraux élastiques (9) opposés a un premier et un deuxième allongement installé, et l'élément de ceinture élastique (15) a un troisième allongement installé, dans lequel les premier et deuxième allongement installés sont sensiblement identiques et sont inférieurs au troisième allongement installé.

4. Article absorbant (1) selon la revendication 3, dans lequel le troisième allongement installé est 20%, de préférence 25%, encore de préférence 30% supérieur au premier allongement installé, encore de préférence dans lequel le troisième allongement installé est de 35% à 80% supérieur au premier allongement installé.

5. Article absorbant (1) selon l'une quelconque des revendications précédentes, dans lequel l'élément de ceinture élastique (15) a un allongement appliqué qui est classé de sorte qu'il varie le long de sa longueur (L).

6. Article absorbant (1) selon les revendications 3 à 5, dans lequel l'élément de ceinture élastique (15) a un allongement appliqué qui est de 15% à 70%, de préférence de 20% à 55% de l'allongement installé.

7. Article absorbant (1) selon l'une quelconque des revendications précédentes, dans lequel l'élément de ceinture élastique (15) est positionné sur l'axe de fixation (14), de sorte que lorsque l'article absorbant (1) est à l'état étendu, l'axe de fixation (14) traverse l'élément de ceinture élastique (15) .

8. Article absorbant (1) selon la revendication 7, dans lequel l'élément de ceinture élastique (15) a un axe central (16) divisant l'élément de ceinture élastique (15) en deux moitiés identiques et s'étendant parallèlement à l'axe transversal (X-X), dans lequel ledit axe central (16) est adjacent à, mais pas aligné avec, l'axe de fixation (14), de préférence dans lequel l'axe central (16) est positionné à une première distance axiale à distance dudit axe de fixation (14) le long de l'axe longitudinal (Y-Y).

9. Article absorbant (1) selon la revendication 8, dans lequel la première distance axiale ne dépasse pas 45%, de préférence 40%, encore de préférence 30%, encore de préférence 25% de la largeur (W).

10. Article absorbant (1) selon l'une quelconque des revendications précédentes, dans lequel les un ou plusieurs canaux sensiblement interconnectés (106) sont symétriques ou asymétriques autour de l'axe longitudinal et de préférence comprend uniquement une partie de canal de raccordement (109) en communication de fluide avec lesdites première et seconde parties de canal (107, 108).

11. Article absorbant (1) selon la revendication 10, dans lequel la partie de canal de raccordement (109) s'étend sensiblement le long de la largeur dudit cœur (8), de préférence formant une forme d'extrémité fermée dans une surface dudit cœur (8) le long d'un plan parallèle à l'axe longitudinal, et de préférence positionnée à l'opposé d'une forme d'extrémité ouverte formée par les première et seconde positions terminales (110, 111) non raccordées des première et seconde parties de canal (107, 108) du canal interconnecté (106) respectivement, lesdites première et seconde positions terminales (110, 111) non raccordées étant distales l'une par rapport à l'autre et à proximité des premier et second côtés (104, 105) dudit cœur (8) respectivement, de préférence lesdites positions terminales (110, 111) étant orientées à l'opposé l'une de l'autre afin de former un espace en forme d'entonnoir entre elles.

12. Article absorbant (1) selon l'une quelconque des revendications précédentes, dans lequel la paire de panneaux latéraux élastiques (9) opposés ont une première et une deuxième résistance élastique, et l'élément de ceinture élastique (15) a une troisième résistance élastique, dans lequel les première et deuxième résistances élastiques sont sensiblement les mêmes et sont supérieures à la troisième résistance élastique.

13. Article absorbant (1) selon l'une quelconque des revendications précédentes, dans lequel la paire de panneaux latéraux élastiques (9) opposés sont assemblés au châssis via un adhésif et/ou par liaison mécanique telle que la liaison par ultrasons.
